# EUROPEAN PATENT APPLICATION

(11) **EP 2 095 831 A1**
(43) Date of publication of application: **02.09.2009**
(21) Application number: 09153714.2
(22) Date of filing: 26.02.2009
(51) Int. Cl.: A61L 27/26, A61L 27/54

(54) **Hydrogel Composition**

(30) Priority: 28.02.2008 US 39546
(71) Applicant: DePuy Products, Inc., Warsaw, IN 46581 (US)
(72) Inventor: King, Richard S, Warsaw, IN 46580 (US)
(74) Representative: Belcher, Simon James

(57) **Abstract**

A hydrogel composition comprises water, a first crosslinked polymer, and a second crosslinked polymer, in which the first crosslinked polymer comprises poly(ethylene oxide) and the second crosslinked polymer comprises a hydrophilic polymer other than poly(ethylene oxide), and in which the first crosslinked polymer and the second crosslinked polymer form an interpenetrating network (IPN). The hydrogel composition can be used as a drug delivery device.

## Description

This invention relates to a hydrogel composition and to method for making such a composition.

The term "hydrogel" is used to refer to a class of polymeric materials which are extensively swollen in an aqueous medium but which do not dissolve in them. Hydrogels have been proposed for a number of applications such as contact lenses, medical implant materials, stents, sutures, sustained or controlled release compositions, bandages, wound dressings, and cell growth matrices.

A number of polymeric materials have been considered for preparing hydrogel. In particular, polyethylene oxide has been proposed as a starting material for preparing hydrogels. For example US-A-2006/0074182 discloses a hydrogel composition comprising crosslinked polyethylene oxide and another hydrophilic polymer dispersed within the crosslinked polyethylene oxide network. The hydrophilic polymer is not crosslinked. The hydrogel composition is a semi-interpenetrating network (semi-IPN).

Important characteristics of hydrogel compositions include water content and compressive strength.

In one aspect, the invention provides a hydrogel composition comprising water, a first crosslinked polymer, and a second crosslinked polymer, in which the first crosslinked polymer comprises poly(ethylene oxide) and the second crosslinked polymer comprises a hydrophilic polymer other than poly(ethylene oxide), in which the first crosslinked polymer and the second crosslinked polymer form an interpenetrating network (IPN).

The hydrogel of the present invention has an attractive combination of water absorption and compressive strength, which makes the hydrogel composition suitable for use in demanding applications such as human joints. The hydrogel composition is elastic and tough (or ductile). The invention also provides a method of preparing such hydrogel composition.

Any suitable poly(ethylene oxide) (PEO) can be used as a precursor to the first crosslinked polymer, including for example one which can be melt-processed. Preferably, the PEO can be crosslinked, for example as a result of exposure to radiation, and especially can be melt-processed and crosslinked. The term "melt-processable" is used to refer to a polymer which can be processed in its molten state using processes such as injection molding, extrusion, blow molding, and/or compression molding. Preferably, a melt-processable polymer does not exhibit significant oxidative degradation, decomposition, or pyrolysis at the temperatures typically used in such moulding processes. The term "radiation crosslinkable" is used to refer to a polymer that forms crosslinks between individual polymer molecules and/or between segments of the same polymer molecule when the polymer is exposed to a suitable amount of radiation, such as gamma, x-ray, or electron beam radiation.

Any suitable PEO can be employed to create the crosslinked PEO. Preferably, the poly(ethylene oxide) has an average molecular weight, prior to crosslinking, of about 400,000 atomic mass units or more, for example about 500,000 atomic mass units or more, about 1,000,000 atomic mass units or more (for example about 2,000,000 atomic mass units or more, about 3,000,000 atomic mass units or more, about 4,000,000 atomic mass units or more, or even about 7,000,000 atomic mass units or more).

PEOs are different from polyethylene glycols in molecular weight. The term PEO refers to the high molecular weight material, i.e., one having a molecular weight of 50,000 or more.

The hydrogel composition of the invention comprises a second crosslinked polymer, which is preferably less hydrophilic than PEO. Any suitable hydrophilic polymer can be present as the second polymer, for example those selected from the group consisting of polyhydroxyethylmethacrylate (PHEMA), polyvinylpyrrolidone (PVP), polyvinyl alcohol, polyacrylic acid, polymethacrylic acid, polyhydroxyethylacrylate, poly(2-aminoethyl) methacrylate, and polyacrylamide, preferably the second crosslinked polymer comprises a hydrophilic polymer selected from the group consisting of polyhydroxyethylmethacrylate (PHEMA) and polyvinylpyrrolidone (PVP). In an embodiment, the second hydrophilic polymer is not kappa-carrageenan.

The hydrogel composition of the invention has a water content less than about 70% or more by weight, for example about 20% to about 60% by weight, about 25% to about 55% by weight, about 30% to about 50% by weight of the composition at 20 °C and 101.3 kPa (1 atmosphere) pressure. The water present in the hydrogel composition is preferably purified water such as water prepared by reverse osmosis (RO water), deionised water, distilled water, and/or water treated with adsorbents such as activated carbon.

The invention also provides a drug delivery device comprising a hydrogel composition and an active ingredient dispersed or dissolved in the hydrogel composition. The drug delivery device can be any suitable device, for example a medical implant, a wound dressing or a transdermal patch, cartilage repair and replacement, and nucleus pulposus replacement.

The invention further provides a method for producing a hydrogel composition comprising water, a first crosslinked polymer, and a second crosslinked polymer, in which the first crosslinked polymer comprises poly(ethylene oxide) and the second crosslinked polymer comprises a hydrophilic polymer other than poly(ethylene oxide), in which the first crosslinked polymer and the second crosslinked polymer form an interpenetrating network (IPN), said method comprising:
(a) providing a compression mould having an internal volume;
(b) filling at least a portion of the internal volume of the compression mould with polyethylene oxide;
(c) heat-compacting the polyethylene oxide within the compression mould to form a consolidated body therefrom;
(d) irradiating at least a portion of the consolidated body to crosslink at least a portion of the polyethylene oxide contained within the consolidated body;
(e) hydrating the consolidated body from (d);
(f) drying the hydrated consolidated body from (e) by lyophilisation, by vacuum, or by heat/vacuum treatment to obtain a foam structure;
(g) imbibing the foam structure from (f) with a composition comprising water and/or polar solvent, a monomer corresponding to the second crosslinked polymer and a hydrophilic crosslinking agent to obtain a composite body;
(h) polymerising the monomer and the hydrophilic crosslinking agent contained in the composite body; and
(i) hydrating the composite body from (h) to obtain the hydrogel composition.

As noted above, at least a portion of the internal volume of the compression mould (the mould cavity) is filled with the PEO. After at least a portion of the internal volume of the compression mould is filled, the PEO contained within the compression mould is compressed for a time and under conditions (for example pressure and temperature) sufficient to form a consolidated body therefrom. It will be understood that the PEO is compressed by any suitable means, such as by mating the two halves of a two-part compression mould and applying an external force in a direction such that any substance contained within the mould (for example the PEO) is subjected to a compressive force.

Typically, the PEO is subjected to a pressure of about 3,400 kPa to about 28,000 kPa during the compression moulding step. Preferably, the PEO is subjected to a pressure of about 3,800 kPa to about 14,000 kPa. During the compression moulding step, the PEO typically is subjected to a temperature of about 70°C to about 200°C. Preferably, the PEO is subjected to a temperature of about 90°C to about 180°C, more preferably a temperature of about 120°C to about 140°C, during the compression moulding step. The PEO can be compressed in the compression mould for any amount of time sufficient to form a consolidated body therefrom. Typically, the PEO is compressed for about 5 to about 30 minutes, more preferably about 5 to about 10 minutes, during the compression moulding step. It will also be understood that the particular time and conditions (for example pressure and temperature) necessary to form a consolidated body from the PEO will depend upon several factors, such as the type and/or amount of PEO, and the size (for example thickness) of the desired consolidated body, as well as molecular weight.

In accordance with an embodiment of the invention, the consolidated body is vacuum foil packaged and irradiated in (d) by exposing it gamma radiation, preferably with a dose of about 15 to about 100 KGy, more preferably about 40 to about 60 KGy.

The consolidated body produced during the compression step can be irradiated using any suitable method, many of which are known in the art. For example the consolidated body can be irradiated by exposing the mass to a suitable amount of gamma, x-ray, or electron beam radiation. Preferably, the consolidated body is irradiated by exposing it to about 15 to about 100 KGy of gamma radiation.

Preferably, the consolidated body is irradiated in an inert or reduced-pressure atmosphere. Irradiating the consolidated body in an inert (i.e., non-oxidising) or reduced-pressure atmosphere reduces the effects of oxidation and chain scission reactions which can occur during irradiation in an oxidative atmosphere. Typically, the consolidated body is placed in an oxygen-impermeable package during the irradiation step. Suitable oxygen-impermeable packaging materials include, but are not limited to, aluminum, polyester coated metal foil (for example the product available from DuPont Teijin Films under the trade mark Mylar), and multi-layer packages comprising polyethylene terephthalate and/or poly(ethylene vinyl alcohol). In order to reduce further the amount of oxidation which occurs during the irradiation of the consolidated body, the oxygen-impermeable packaging may be evacuated (for example the pressure within the packaging may be reduced below the ambient atmospheric pressure) and/or flushed with an inert gas (for example nitrogen, argon, helium, or mixtures thereof) after the consolidated body has been placed in the packaging.

After the irradiation, the consolidated body is exposed to water to obtain a hydrated PEO gel. The consolidated body can be hydrated by any suitable technique. Typically, at least a portion of the consolidated body is submerged in an aqueous solution (for example de-ionised water, water filtered by reverse osmosis, a saline solution, or an aqueous solution containing a suitable active ingredient) for an amount of time sufficient to produce a hydrogel having the desired water content. For example when a hydrogel comprising the maximum water content is desired, the consolidated body is submerged in the aqueous solution for an amount of time sufficient to allow the consolidated body to swell to its maximum size or volume. Typically, the consolidated body is submerged in the aqueous solution for at least about 50 hours, preferably at least about 100 hours, and more preferably about 120 hours to about 240 hours (for example about 120 hours to about 220 hours). The aqueous solution used to hydrate the consolidated body can be maintained at any suitable temperature. Typically, the aqueous solution is maintained at a temperature of at least about 25°C, more preferably at a temperature of about 30°C to about 100°C, and most preferably at a temperature of about 40°C to about 90°C (for example about 50°C to about 90°C, or about 50°C to about 80°C).

The hydrated PEO gel is dried by lyophilisation, vacuum drying, or heat/vacuum drying to obtain a dry gel. The dry gel is immersed in an aqueous composition comprising water, optionally with a polar solvent, a monomer, and a crosslinking agent. The monomer can be present in any suitable concentration, for example about 40% or more, such as from about 50% to about 80%, preferably from about 55% to about 75%, by weight of the composition. The crosslinking agent can be present in any suitable concentration, for example about 1 ppm or more, such as from about 1 ppm to about 100 ppm, preferably from about 5 ppm to about 10 ppm by weight of the composition.

The monomer corresponding to the second crosslinked hydrophilic polymer is selected from the group consisting of 2-hydroxyethyl methacrylate, N-vinyl pyrrolidone, 2-aminoethyl methacrylate, and acrylamide, 2-hydroxybutyl methacrylate, 4-hydroxybutyl methacrylate, glycidyl methacrylate, glyceryl methacrylate, propylene glycol monomethacrylate, ethoxyethyl methacrylate, triethylene glycol methacrylate, 4- or 2-vinyl pyridine, and N, N-dimethyl acrylamide.

In accordance with an embodiment, the hydrophilic crosslinking agent is a bifunctional crosslinking agent, trifunctional crosslinking agent, or a tetrafunctional crosslinking agent. For example the hydrophilic crosslinking agent is selected from the group consisting of N,N-methylene bisacrylamide, diethylene glycol dimethacrylate, triethylene glycol dimethacrylate, diethylene glycol acrylate, triethylene glycol acrylate, polyethylene glycol diacrylate, and polyethylene glycol dimethacrylate.

In accordance with an embodiment, the imbibed PEO gel is vacuum foil packaged and the monomer and the hydrophilic crosslinking agent are polymerised by exposing the composite body from (e) to about 15 to about 100 KGy, preferably about 40 to about 60 KGy, of gamma radiation. The radiation can be from a radioactive isotope such as ⁶⁰Co (gamma rays).

In accordance with another embodiment of the invention, the consolidated body in (g) further includes a free radical initiator. Any suitable free radical initiator can be included, for example a peroxide initiator such as benzoyl peroxide or dicumyl peroxide, or an azo initiator such as AIBN. The monomer and the crosslinking agent can be polymerised by heating the composite body from (g).

Subsequent to the polymerisation of the monomer and the crosslinking agent, the composite body in (h) is hydrated by submerging at least a portion of the composite body in an aqueous medium, which can include, in addition to water, additives such as therapeutic agents, humectants, salts, and/or surfactants. The invention also provides a hydrogel composition produced by the method described above.

The hydrogel composition of the invention can also contain any suitable additive, such as a therapeutic agent, surfactant, or humectant. The therapeutic agent can be a small molecule or a macromolecule. Examples of therapeutic agents include antiinfectives such as antibiotics and antiviral agents; analgesics and analgesic combinations; anorexics; antihelmintics; antiarthritics; antiasthmatic agents; anticonvulsants; antidepressants; antidiuretic agents; antidiarrheals; antihistamines; antiinflammatory agents; antimigraine preparations; antinauseants; antineoplastics; antiparkinsonism drugs; antipruritics; antipsychotics; antipyretics, antispasmodics; anticholinergics; sympathomimetics; xanthine derivatives; cardiovascular preparations including calcium channel blockers and beta-blockers such as pindolol and antiarrhythmics; antihypertensives; diuretics; vasodilators including general coronary, peripheral and cerebral; central nervous system stimulants; cough and cold preparations, including decongestants; hormones such as estradiol and other steroids, including corticosteroids; hypnotics; immunosuppressives; muscle relaxants; parasympatholytics; psychostimulants; sedatives; and tranquilers; and naturally derived or genetically engineered proteins, polysaccharides, glycoproteins, or lipoproteins, particularly bone growth factors and bone proteins.

Any suitable biocompatible surface active agent can be employed. Examples of surface active agents include phospholipids. Any suitable biocompatible humectants can be employed. Examples of suitable humectants include glycerin, propylene glycol, polyethylene glycol, glycol ethers such as diethylene glycol monomethyl ether and propylene glycol monomethyl ether.

The additive can be incorporated into the hydrogel composition by any suitable technique. For example the additive can be incorporated (for example dry blended) into the PEO which is used to produce the hydrogel composition. Alternatively, the additive can be incorporated into the hydrogel composition during the hydration step, particularly the second hydration step. In particular, the hydrogel can be hydrated using an aqueous and/or organic solution containing the additive. Alternatively, a hydrogel can be dehydrated or dried to produce a xerogel, and then the xerogel can be hydrated using an aqueous and/or organic solution containing the additive.

The hydrogel composition of the invention can have a compressive strength greater than 1 MPa. In a preferred embodiment, the hydrogel composition of the invention has a compressive strength of about 1.5 MPa or more. In a more preferred embodiment, the hydrogel composition of the invention has a compressive strength of about 2 MPa or more, most preferably 2.1 to about 3.0 MPa. In accordance with an embodiment, the hydrogel composition has a water content of about 40% to about 50% and a compressive strength of 2 to about 2.6 MPa.

The term "compressive strength" is used to refer to the maximum resistance to fracture of a material (for example the hydrogel composition) under compressive stress. The compressive strength of the hydrogel composition can be measured using any suitable technique. One suitable technique for determining the compressive strength of a hydrogel is the method of C Tranquilan-Aranilla *et al,* which is described in the article "Kappa-carrageenan-polyethylene oxide hydrogel blends prepared by gamma irradiation", Radiation Physics and Chemistry 55:127-131 (1999). Preferably, the compressive strength of the hydrogel composition is determined using a modified version of the technique developed by C Tranquilan-Aranilla *et al* in which the sample used for the test is changed to measure approximately 15.9 mm (0.625 inch) in diameter and about 6.4 mm (0.25 inch) in thickness. The crosshead speed used in the modified technique is approximately 10 mm.min⁻¹ (0.4 inch.min⁻¹), which is the same crosshead speed used by C Tranquilan-Aranilla *et al.* The compressive strength of a hydrogel is considered to be within the ranges set forth herein when determined using the aforementioned preferred technique (i.e., the modified technique based upon the method of C Tranquilan-Aranilla *et al*).

The method of the invention can include the step of sterilising the hydrogel composition using any suitable process. The hydrogel composition can be sterilised at any suitable point, but preferably is sterilised after the composite body/hydrogel precursor is hydrated. Suitable non-irradiation sterilisation techniques include, but are not limited to, gas plasma or ethylene oxide methods known in the art. For example the hydrogel composition can be sterilised using a sterilisation system such as that sold under the trade mark PlazLyte by Abtox Inc or using the gas plasma sterilisation processes disclosed in US-5413760 and US-5603895. In some circumstances, such as when the interior portions of the hydrogel composition require sterilisation, the hydrogel composition can be sterilised using gamma irradiation at a relatively low dose of approximately 15 KGy to about 40 KGy using conventional methods.

The hydrogel composition produced by the method of the invention can be packaged in any suitable packaging material. Desirably, the packaging material maintains the sterility of the hydrogel composition until the packaging material is breached.

### EXAMPLE 1

This example demonstrates the production of a hydrogel composition according to the invention.

A poly(ethylene oxide) having an average molecular weight of approximately 7 million atomic mass units (sold by The Dow Chemical Company, Midland, Michigan under the trade mark POLYOX WSR-303) is placed into a compression mold having an internal volume. The internal volume of the compression mold defines a disk having a diameter of approximately 89 mm (3.5 inch) and a thickness of approximately 3.2 mm (0.125 inch), and the PEO completely fills the compression mold. The PEO is packed into the compression mold by subjecting the consolidated body to a compressive force of approximately 2700 kPa (400 psi) for approximately 2 to 3 minutes at room temperature. Following the packing step, the temperature within the compression mold is increased from room temperature to approximately 127°C (260°F) at a rate of approximately 3.3°C.min⁻¹ (6°F.min⁻¹) while the pressure on the compression mold is increased from 2700 kPa (400 psi) to about 11,000 kPa (1600 psi). The temperature and pressure within the compression mold are then maintained at approximately 127°C (260°F) and 11,000 kPa (1600 psi) for approximately 15 minutes. Following the heat-compacting step, the resulting consolidated disk is cooled from 127°C (260°F) to room temperature (i.e., approximately 22°C (72°F)) at rate of approximately 3.9°C.min⁻¹ (7°F.min⁻¹) while the pressure is reduced from 11,000 kPa (1600 psi) to approximately 480 kPa (70 psi).

The consolidated disk is vacuum packed in a foil, and is subjected to gamma irradiation to a dose of 50 KGy. The irradiated disk is immersed in water for 72 hours or longer at room temperature. The hydrated disk is dried by lyophilisation to obtain a dry PEO gel. The dry PEO gel is soaked in an aqueous solution containing a hydrophilic monomer and a crosslinking agent. The PEO gel soaked with the aqueous solution is packaged in vacuum foil and gamma irradiated to a dose of 50 KGy. The irradiated gel is soaked in water to obtain a hydrated gel, which is later imbibed with a monomer and crosslinking agent, followed by irradiation, as set forth below.

The table below sets out the properties of three PEO IPNs in accordance with the invention. The IPNs have attractive properties such as high compressive strength and high compressive modulus while maintaining water absorption.

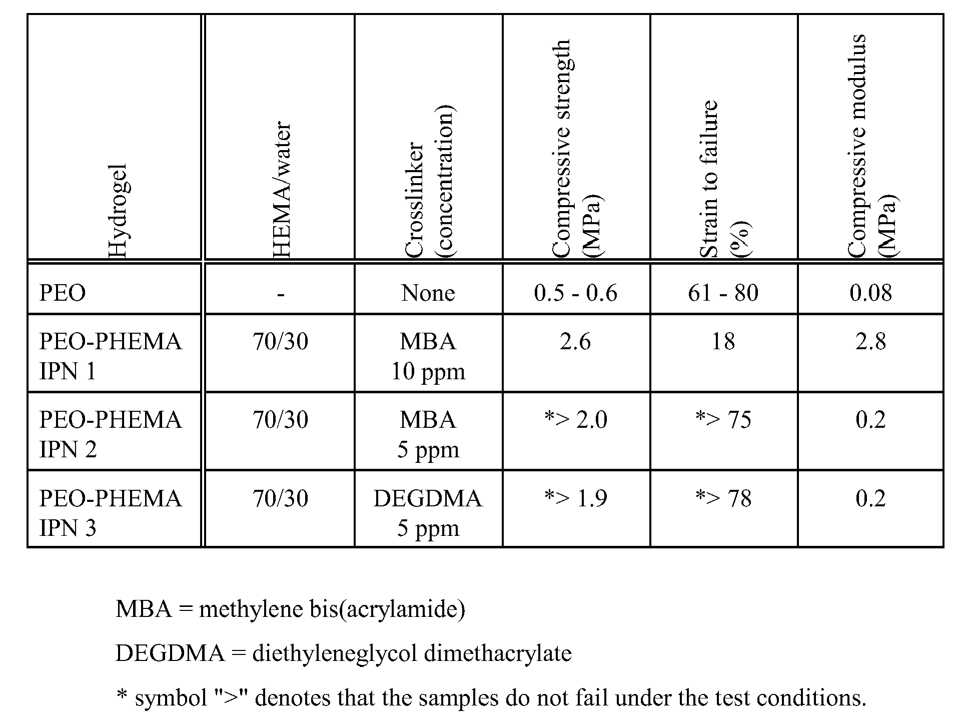

### EXAMPLE 2

This Example illustrates the water content of hydrogels prepared in accordance with another embodiment of the invention.

The water content of PEO crosslinked by 50 KGy gamma radiation is 93.4%.

The water content of IPN polyethylene oxide - polyhydroxyethyl methacrylate (in which pHEMA and water (but no crosslinking agent) are present in the proportions 70:30) is 51.7%. The semi-IPN hydrogel was fabricated using the same procedure as IPN hydrogels described above except for the absence of a crosslinking agent. Polymerisation is initiated by 50 KGy gamma treatment.

The water content of IPN polyethylene oxide - polyhydroxyethyl methacrylate (in which pHEMA, water and N,N-methylene bisacrylamide are present in the proportions 70:30:0.001) is 48.2%. Polymerisation and crosslinking of PHEMA are initiated by radiation treatment at 50 KGy.

The water content of IPN poly(ethylene oxide) - polyhydroxyethyl methacrylate (in which pHEMA, water and diethylene glycol dimethacrylate are present in the proportions 70:30:0.001) is 43.2%. Polymerisation and crosslinking of pHEMA are initiated by radiation treatment at 50 KGy.

## Claims

1. A hydrogel composition comprising water, a first crosslinked polymer, and a second crosslinked polymer, in which the first crosslinked polymer comprises poly(ethylene oxide) and the second crosslinked polymer comprises a hydrophilic polymer other than poly(ethylene oxide), in which the first crosslinked polymer and the second crosslinked polymer form an interpenetrating network (IPN).

2. A composition as claimed in claim 1, in which the second crosslinked polymer comprises a hydrophilic polymer selected from the group consisting of polyhydroxyethylmethacrylate (PHEMA), polyvinylpyrrolidone (PVP), polyvinyl alcohol, polyacrylic acid, polymethacrylic acid, polyhydroxyethylacrylate, poly(2-aminoethyl) methacrylate, and polyacrylamide.

3. A composition as claimed in claim 1, which has a water content less than about 70%, preferably from about 20% to about 60%, by weight at 20°C and 101.3 kPa (1 atmosphere) pressure.

4. A drug delivery device comprising a hydrogel composition as claimed in any one of claims 1 to 3 and an active ingredient dispersed or dissolved in the hydrogel composition.

5. A device as claimed in claim 4, which is a wound dressing.

6. A method of producing a hydrogel composition comprising water, a first crosslinked polymer, and a second crosslinked polymer, in which the first crosslinked polymer comprises poly(ethylene oxide) and the second crosslinked polymer comprises a hydrophilic polymer other than poly(ethylene oxide), in which the first crosslinked polymer and the second crosslinked polymer form an interpenetrating network (IPN), said method comprising:
(a) providing a compression mould having an internal volume;
(b) filling at least a portion of the internal volume of the compression mould with polyethylene oxide;
(c) heat-compacting the polyethylene oxide within the compression mould to form a consolidated body therefrom;
(d) irradiating at least a portion of the consolidated body to crosslink at least a portion of the polyethylene oxide contained within the consolidated body;
(e) hydrating the consolidated body from (d);
(f) drying the hydrated consolidated body from (e) by lyophilisation, vacuum, or heat/vacuum to obtain a foam structure;
(g) imbibing the foam structure from (f) with a composition comprising water and/or polar solvent, a monomer corresponding to the second crosslinked polymer and a hydrophilic crosslinking agent to obtain a composite body;
(h) polymerising the monomer and the hydrophilic crosslinking agent contained in the composite body; and
(i) hydrating the composite body from (h) to obtain the hydrogel composition.

7. A method as claimed in claim 6, in which the polyethylene oxide has a molecular weight of about 1,000,000 or more.

8. A method as claimed in claim 6, in which polyethylene oxide is compressed at a pressure of about 3,800 kPa to about 14,000 kPa.

9. A method as claimed in claim 6, in which the polyethylene oxide is heated to a temperature of about 120°C to about 140°C during the compression.

10. A method as claimed in claim 6, in which the consolidated body is irradiated in (d) by exposing it to about 15 to about 100 KGy of gamma radiation.

11. A method as claimed in claim 6, in which the monomer in (g) is selected from the group consisting of hydroxyethyl methacrylate, N-vinyl pyrrolidone, 2-aminoethyl methacrylate, and acrylamide.

12. A method as claimed in claim 6, in which the hydrophilic crosslinking agent is selected from the group consisting of N,N-methylene bisacrylamide, diethylene glycol dimethacrylate, triethylene glycol dimethacrylate, diethylene glycol acrylate, triethylene glycol acrylate, polyethylene glycol diacrylate, polyethylene glycol dimethacrylate.

13. A method as claimed in claim 6, in which the monomer and the hydrophilic crosslinking agent are polymerised by exposing the moulded body from (e) to about 15 to about 100 KGy of gamma radiation.

14. A method as claimed in claim 6, in which the consolidated body in (g) further includes a free radical initiator.

15. A method as claimed in claim 14, in which monomer and the crosslinking agent are polymerised by heating the composite body from (g).
